(19) **Europäisches Patentamt**
**European Patent Office**
**Office européen des brevets**

(11) **EP 4 424 241 B1**

(12) **EUROPEAN PATENT SPECIFICATION**

(45) Date of publication and mention
of the grant of the patent:
**22.04.2026  Bulletin 2026/17**

(21) Application number: **22887154.7**

(22) Date of filing: **28.10.2022**

(51) International Patent Classification (IPC):
*A61B 5/11* (2006.01)  *G06T 7/00* (2017.01)
*G06T 7/20* (2017.01)

(52) Cooperative Patent Classification (CPC):
**G06T 7/00; A61B 5/00; A61B 5/11; A61B 5/1128;
A61B 5/16; A61B 5/4082; A61B 5/4088;
A61B 5/7267; G06T 7/20**

(86) International application number:
**PCT/JP2022/040289**

(87) International publication number:
**WO 2023/074829 (04.05.2023 Gazette 2023/18)**

(54) **COGNITIVE FUNCTION ASSESSMENT SYSTEM AND TRAINING METHOD**

SYSTEM ZUR BEURTEILUNG KOGNITIVER FUNKTIONEN UND TRAININGSVERFAHREN

SYSTÈME D'ÉVALUATION DE FONCTION COGNITIVE ET PROCÉDÉ DE FORMATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.10.2021  JP 2021177748**

(43) Date of publication of application:
**04.09.2024  Bulletin 2024/36**

(73) Proprietor: **OSAKA UNIVERSITY
Suita-shi
Osaka 565-0871 (JP)**

(72) Inventors:
• **YAGI, Yasushi**
  **Suita-shi, Osaka 565-0871 (JP)**
• **WU, Shuqiong**
  **Suita-shi, Osaka 565-0871 (JP)**
• **OKURA, Fumio**
  **Suita-shi, Osaka 565-0871 (JP)**
• **MAKIHARA, Yasushi**
  **Suita-shi, Osaka 565-0871 (JP)**
• **AOKI, Kota**
  **Suita-shi, Osaka 565-0871 (JP)**

(74) Representative: **Viering, Jentschura & Partner
mbB
Patent- und Rechtsanwälte
Am Brauhaus 8
01099 Dresden (DE)**

(56) References cited:
**WO-A1-2016/148199        WO-A1-2016/148199
WO-A1-2021/006522        CN-A- 112 070 027
CN-A- 112 070 027        CN-A- 112 543 936
CN-A- 112 543 936        JP-A- 2021 030 050
JP-A- 2021 521 536        KR-A- 20210 086 881
US-A1- 2015 208 975        US-A1- 2018 078 184
US-A1- 2020 405 216**

• **WU SHUQIONG ET AL: "Early Detection of Low
Cognitive Scores from Dual-task Performance
Data Using a Spatio-temporal Graph
Convolutional Neural Network", 2021 43RD
ANNUAL INTERNATIONAL CONFERENCE OF
THE IEEE ENGINEERING IN MEDICINE &
BIOLOGY SOCIETY (EMBC), IEEE, 1 November
2021 (2021-11-01), pages 1895 - 1901,
XP034041427, [retrieved on 20211129], DOI:
10.1109/EMBC46164.2021.9630304**

**(Cont. next page)**

- **MATUSURA TAKU: "Data collection and analysis of elderly people with dementia using a dual-task experience system", IPSJ SIG TECHNICAL REPORT, vol. 2017, no. 10, 8 November 2017 (2017-11-08), pages 1 - 7, XP093060142**

**Description**

TECHNICAL FIELD

The present invention relates to a cognitive function evaluation system and a learning method.

BACKGROUND ART

Early detection of cognitive decline is important for retarding the progression of dementia. For example, Non-patent Literature 1 discloses a system for early detection of decline in a cognitive function through a dual task. Specifically, the system of Non-patent Literature 1 calculates 12 features related to dementia from data that has been collected by having a subject perform a single task and a dual task. The system then estimates the mini-mental state examination (MMSE) score through machine learning.

Specifically, the system of Non-patent Literature 1 requires a subject to perform a stepping exercise through the single task which is a physical task. The dual task includes a physical task and a cognitive task to be performed simultaneously. The physical task of the dual task requires a subject to perform a stepping exercise like the single task. The cognitive task of the dual task requires the subject to answer calculation questions or "rock paper scissors problems". The system of Non-patent Literature 1 calculates six features below from each of single task data and dual task data (12 features in total):

(1) Average speed of stepping on the spot;
(2) Standard deviation of varying speed of stepping on the spot;
(3) Average knee angle;
(4) Standard deviation of knee angles;
(5) Ratio of correct answers to cognitive task; and
(6) Average number of answers to cognitive task.

Patent Literature 1 discloses a dual-task performing ability evaluation method including a dual task step, an analysis step, and an evaluation step. In the dual task step, a subject performs a dual task including a movement task that requires the subject to perform specific movement and an intelligence task that requires the subject to answer a specific problem. Further, at least one of motion and an answer of the subject performing the dual task is detected. In the analysis step, the at least one of the motion and the answer that has been detected is analyzed. In the evaluation step, dual-task performing ability of the subject is evaluated based on a result of the analysis.

Patent Literature 2 discloses a method that includes prompting a subject to perform a plurality of iterations of a physical task including a movement of a first body part of the subject's body. For each iteration of the plurality of iterations of the physical task, one or more measurements corresponding to the movement of the first body part are obtained. A nominal path of the first body part is determined based on the measurements obtained from a first subset of the plurality of iterations of the physical task. A variability metric is generated by analyzing a plurality of measurements with respect to the nominal path. The variability metric is compared with a predetermined baseline to categorize the cognitive performance of the subject.

Patent Literature 3 discloses a cognitive function evaluation device including: an obtainment unit configured to obtain, as gait data, at least one of first data and second data, the first data indicating a sway amount of a body of a subject during walking in a first walking section from start of walking of the subject to a predetermined number of steps, and the second data indicating a sway amount of the body of the subject during walking in a second walking section in a double task state in which the subject is walking while doing a given assignment, the second walking section being after the first walking section; a calculation unit configured to calculate a feature value based on the gait data; an evaluation unit configured to evaluate a cognitive function of the subject, based on the feature value; and an output unit configured to output a result of evaluation by the evaluation unit.

Patent Literature 4 discloses a method comprising: updating model parameters of a pre-training model by utilizing a first sequence data set of a human skeleton point sequence and a visual angle label corresponding to each piece of first sequence data in the first sequence data set; initializing model parameters of a human body action recognition model based on the updated model parameters of the pre-trained model; wherein the pre-training model and the human body action recognition model have feature extraction networks with the same structure; and updating model parameters of the human body action recognition model by utilizing the second sequence data set of the human body skeleton point sequence and the action category label corresponding to each second sequence data in the second sequence data set to obtain a trained human body action recognition model.

CITATION LIST

Non-patent Literature

Non-patent Literature 1: Taku Matsuura and six others, "Cognitive function score estimation for elderly people based on dual-task gait analysis", IECE, Technical Research Report, Kawasaki, Vol. 119, No. HCS2019-99, pp. 83-88, Mar. 2020.

Patent Literature

Patent Literature 1: US 2018/078184 A1; Patent Literature 2: US 2015/208975 A1; Patent Literature 3: US 2020/405216 A1; Patent Literature 4: CN 112 070 027 A

SUMMARY OF INVENTION

Technical Problem

The system of Non-patent Literature 1 is however low in accuracy due to cognitive function score estimation based on limited features. There is therefore room for improvement.
The present invention has been achieved in view of the above circumstances, and an object thereof is to provide a cognitive function evaluation system and a learning method, capable of more accurately evaluate a subject's cognitive function.

Solution to Problem

In an aspect of the present invention, a cognitive function evaluation system includes a motion detector, an answer detector, and an evaluator. The motion detector captures images of a subject performing a predetermined task to generate frames representing three-dimensional coordinates of all joints of the subject whose images have been captured. The frames are a series of frames generated in time order. The answer detector detects answers to questions on a predetermined cognitive examination by the subject performing the predetermined task. The evaluator outputs motion features based on the frames and evaluates a cognitive function of the subject based on the motion features and the answers detected by the answer detector. The motion features represent a feature of a spatial positional relationship of all the joints and a feature of temporal variations of each of the joints. The predetermined task includes a physical task that requires the subject to perform a predetermined behavior, and a cognitive task that requires the subject to answer the questions on the predetermined cognitive examination. The motion detector captures the images of the subject performing the physical task to generate the frames.
In an embodiment, the evaluator classifies the cognitive function of the subject into a class in which a cognitive function score indicating a cognitive ability of the subject is less than or equal to a threshold or a class in which the cognitive function score is greater than the threshold.
In an embodiment, according to the threshold that is set in advance, the evaluator classifies the subject into a class of dementia or a class of mild cognitive impairment and non-dementia, or into a class of dementia and mild cognitive impairment or a class of non-dementia.
In an embodiment, the evaluator determines a cognitive function score indicating a cognitive ability of the subject.
In an embodiment, the evaluator classifies the subject into a class of dementia, a class of mild cognitive impairment, or a class of non-dementia.
In an embodiment, the evaluator classifies the subject into any one of at least two types of the dementia.
In an embodiment, the evaluator includes a motion feature extractor. The motion feature extractor extracts the motion features by: generating respective spatial graphs for the frames, each of the respective spatial graphs indicating respective spatial positional relationships of all the joints; convolving the spatial graphs; generating time graphs across the frames, each of the time graphs representing respective variations in an identical joint between each adjacent frames; and convolving the time graphs.
In an embodiment, the evaluator includes a plurality of motion feature extractors each of which corresponds to the motion feature extractor. Each of the plurality of motion feature extractors is supplied with corresponding frames for each time the predetermined task is performed a plurality of times continuously. The evaluator evaluates the cognitive function of the subject based on the motion features acquired from each of the plurality of motion feature extractors and the answers detected by the answer detector.
In an embodiment, the predetermined task includes a dual task that requires the subject to perform the physical task and the cognitive task simultaneously. The motion detector captures images of the subject performing the dual task. The answer detector detects answers by the subject performing the dual task.
In an aspect of the present invention, a learning method determines parameter values for a neural network that classifies a subject as positive or negative. The learning method includes determining the parameter values through a loss function that optimizes a sum of sensitivity and specificity. The sensitivity describes a rate of the subject being identified as true positive. The specificity describes a rate of the subject being identified as true negative.

In an aspect of the present invention, a learning method determines parameter values for a neural network. The neural network includes a first network and a second network that convolve spatial graphs and convolve time graphs. The spatial graphs represent respective spatial positional relationships of joints of a subject. The time graphs represent respective temporal variations of the joints of the subject. The learning method includes determining parameter values of the first network by learning from data entered into the first network and determining parameter values of the second network by learning from data entered into the second network after setting the determined parameter values of the first network as initial values of parameter values of the second network.

Advantageous Effects of Invention

The cognitive function evaluation system and the learning method according to the present invention are capable of more accurately evaluate a subject's cognitive function.

BRIEF DESCRIPTION OF DRAWINGS

[FIG. 1]

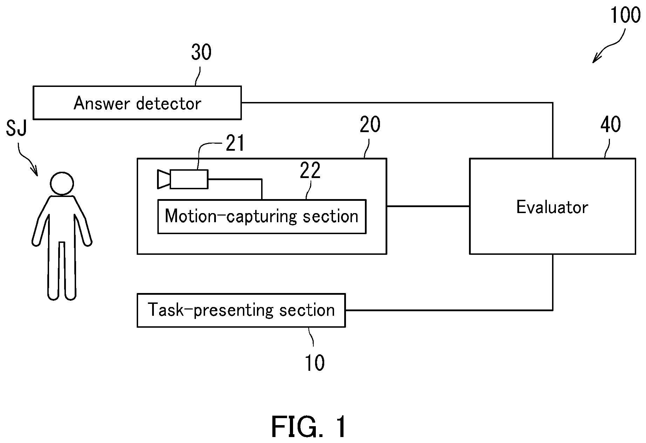

FIG. 1 is a diagram depicting a cognitive function evaluation system according to a first embodiment of the present invention.
[FIG. 2]

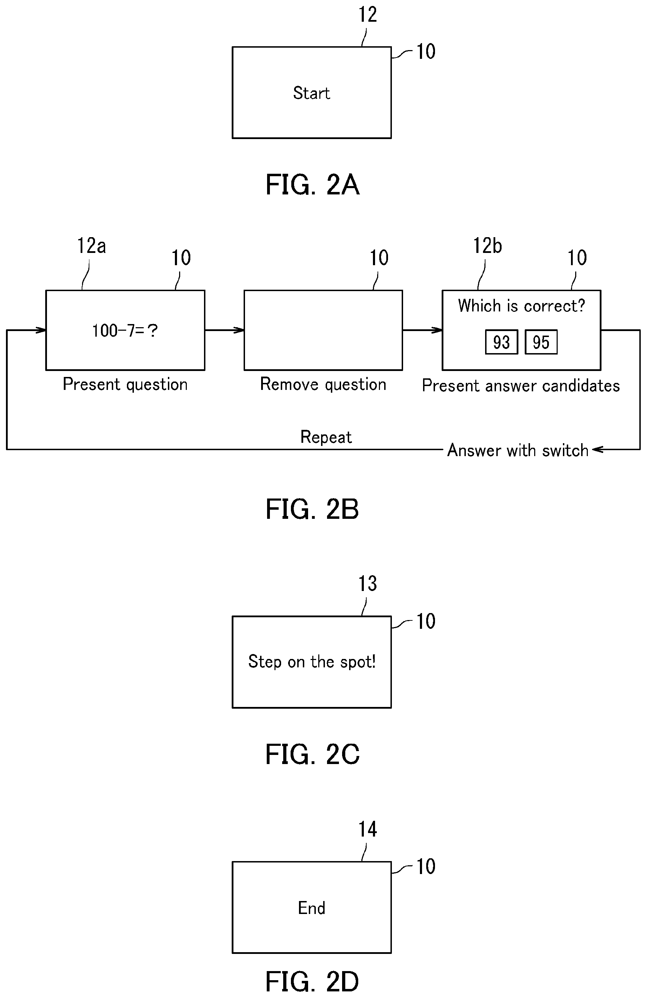

FIGS. 2A to 2D depict an example of a task that a task-presenting section presents to a subject.
[FIG. 3]

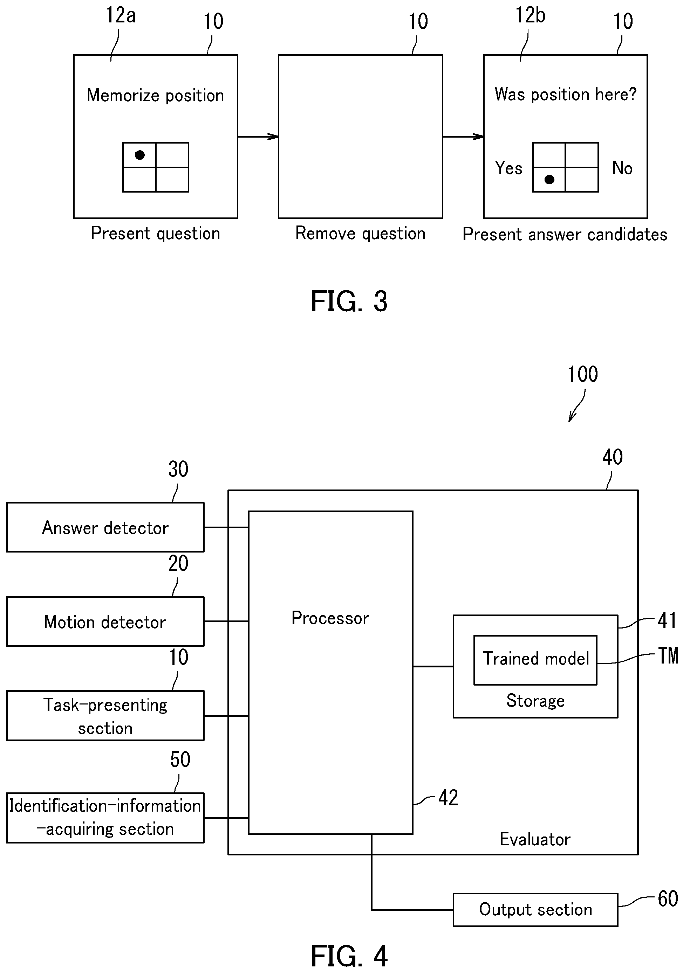

FIG. 3 depicts another example of a question on a cognitive examination that the task-presenting section presents to the subject.
[FIG. 4]
FIG. 4 is a block diagram depicting the configuration of the cognitive function evaluation system according to the first embodiment of the present invention.
[FIG. 5]

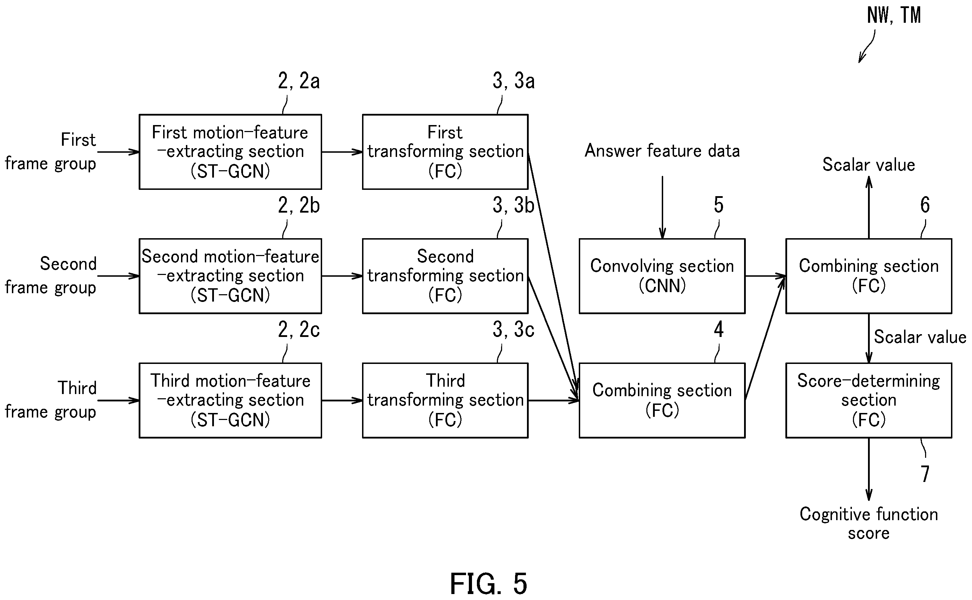

FIG. 5 illustrates a neural network built by a trained model.
[FIG. 6]

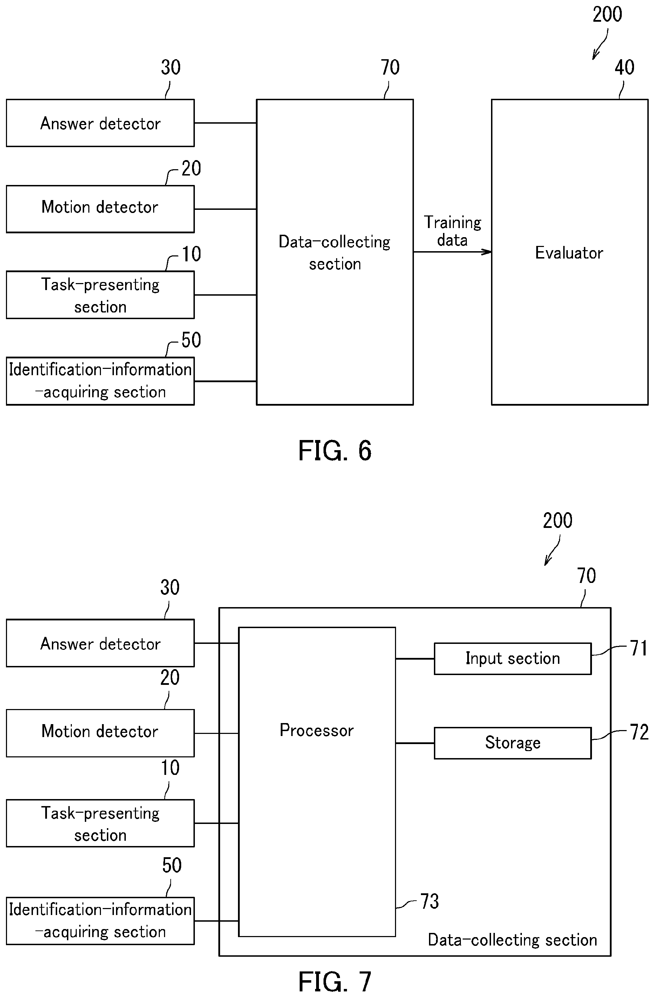

FIG. 6 is a diagram depicting a model-generating system that generates a trained model.
[FIG. 7]
FIG. 7 is a block diagram depicting a part of the configuration of the model-generating system.
[FIG. 8]

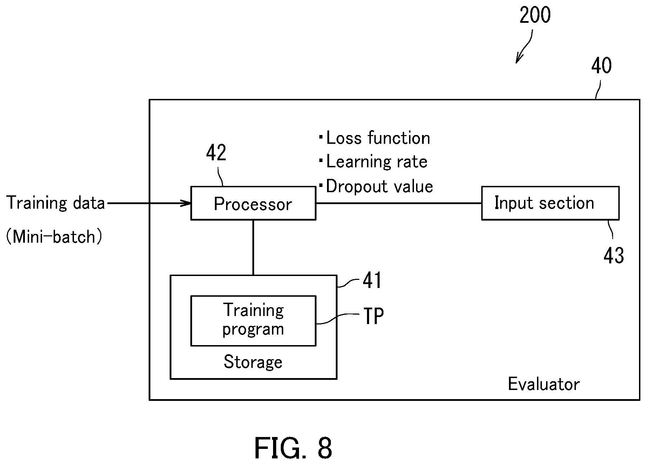

FIG. 8 is a block diagram depicting the configuration of an evaluator during learning.
[FIG. 9]

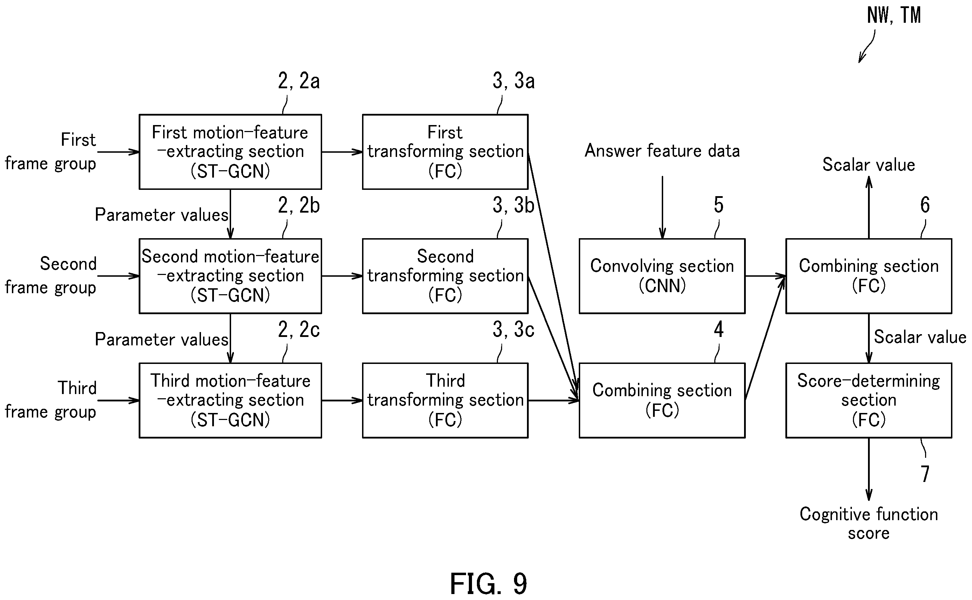

FIG. 9 illustrates a neural network built by a training program.
[FIG. 10]

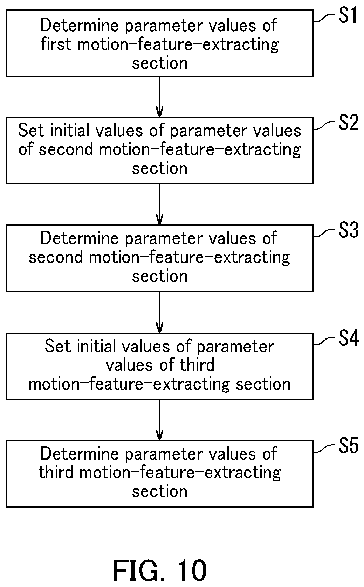

FIG. 10 is a flow chart depicting a learning method according to the first embodiment of the present invention.
[FIG. 11]

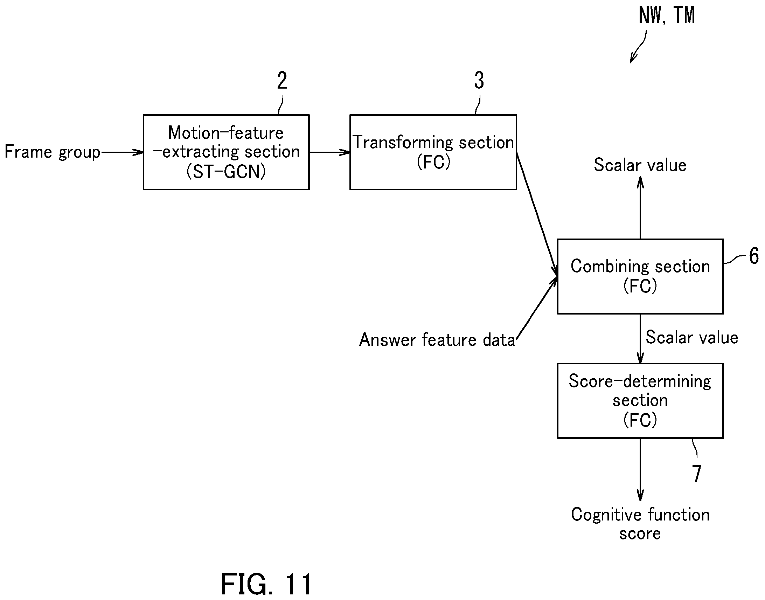

FIG. 11 is a diagram depicting another example of the neural network built by the trained model.
[FIG. 12]

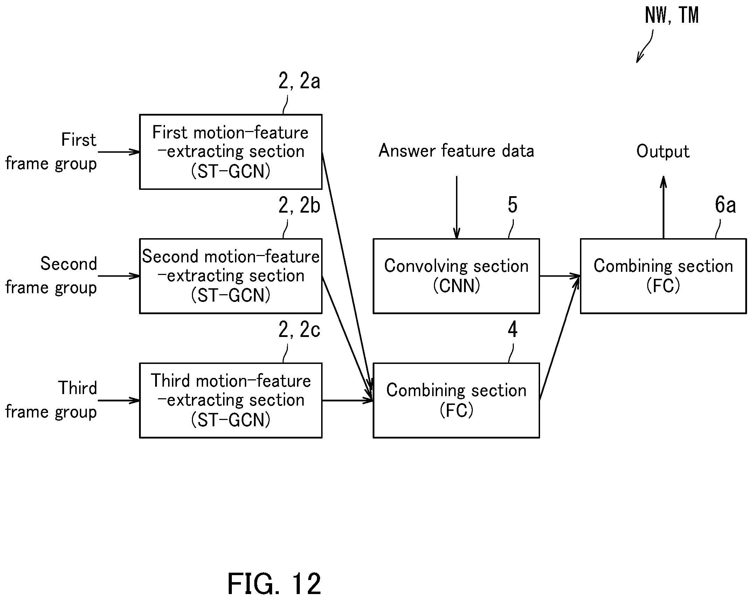

FIG. 12 is a diagram depicting a neural network included in a cognitive function evaluation system according to a second embodiment of the present invention.

DESCRIPTION OF EMBODIMENTS

Embodiments of a cognitive function evaluation system and a learning method of the present invention will be described below with reference to the drawings (FIGS. 1 to 12). However, the present invention is not limited to the following embodiments and may be implemented in various manners within a scope of the invention as defined by the claims. Duplicate descriptions may be omitted as appropriate. Elements that are the same or equivalent are labelled with the same reference signs in the drawings and description thereof is not repeated.

[First Embodiment]

A cognitive function evaluation system 100 according to the present embodiment will first be described with reference to FIG. 1. FIG. 1 is a diagram depicting the cognitive function evaluation system 100 according to the present embodiment. The cognitive function evaluation system 100 according to the present embodiment evaluates a cognitive function of a subject SJ. More specifically, the cognitive function evaluation system 100 according to the present embodiment classifies subjects SJ into a class of dementia (cognitive impairment) or a class of mild cognitive impairment (MCI) and non-dementia. Alternatively, the cognitive function evaluation system 100 according to the present embodiment classifies subjects SJ into a class of dementia and mild cognitive impairment or a class of non-dementia. The cognitive function evaluation system 100 according to the present embodiment further determines a cognitive function score of a subject SJ. The cognitive function score indicates a cognitive ability of the subject SJ.

Note that the mild cognitive impairment is in a prodromal stage of dementia and occurs as an intermediate state between a normal state and a dementia state. Specifically, the mild cognitive impairment involves a decline in a cognitive function such as memory and attention, but the decline is not large enough to interfere with daily life. The cognitive function score is, for example, a general intellectual evaluation scale such as the mini-mental state examination (MMSE) score or Hasegawa dementia scale. In diagnosis using MMSE scores, it is determined that dementia is suspected if the MMSE score is less than or equal to 23 points, while mild cognitive impairment is suspected if the MMSE score is greater than 23 points and less than or equal to 27 points. It is further determined that the subject is considered non-dementia if the MMSE score is greater than 27 points.

As depicted in FIG. 1, the cognitive function evaluation system 100 according to the present embodiment includes a task-presenting section 10. The task-presenting section 10 presents tasks to be performed by a subject SJ. In the present embodiment, the task-presenting section 10 includes a display such as a liquid crystal display. The task-presenting section 10 causes the display to present a screen showing a task to be performed by a subject SJ. Note that the display is placed in front of the subject SJ, for example.

In the present embodiment, tasks to be performed by a subject SJ include a dual task. The dual task is a physical task and a cognitive task to be simultaneously performed by a subject SJ. The physical task requires the subject SJ to perform a predetermined behavior. The cognitive task requires the subject SJ to answer questions on a predetermined cognitive examination. Examples of the predetermined behavior include, on the spot, stepping, walking, running, and skipping. Examples of the questions on the predetermined cognitive examination include calculation problems, location memory problems, and rock paper scissors problems.

In the present embodiment, the tasks to be performed by a subject SJ include a cognitive task (a single task), a physical task (a single task), and the dual task. That is, the subject SJ is to perform the cognitive task, the physical task, and the dual task successively in this order.

More specifically, the cognitive task (single task) is performed for a predetermined time (e.g., 30 seconds) by a subject SJ. The physical task (single task) is then performed for a predetermined time (e.g., 20 seconds) by the subject SJ. The dual task is finally performed for a predetermined time (e.g., 30 seconds) by the subject SJ. The time taken for the subject SJ to perform the cognitive task (single task) is also hereinafter referred to as "cognitive task performance time". Similarly, the time taken for subject SJ to perform the physical task (single task) is also hereinafter referred to as "physical task performance time". In addition, the time taken for the subject SJ to perform the dual task is also hereinafter referred to as "dual task performance time". Note that the cognitive task performance time is arbitrary in length. Similarly, the physical task performance time and the dual task performance time are also arbitrary in length.

In the present embodiment, the questions on the cognitive examination presented to the subject SJ through the cognitive task (single task) are the same as the questions on the cognitive examination of the cognitive task included in the dual task. The behavior required for the subject SJ through the physical task (single task) is the same as the behavior of the physical task included in the dual task. Note that the questions on the cognitive examination presented to the subject SJ through the cognitive task (single task) may be different from that of the cognitive task included in the dual task. Similarly, the behavior required for the subject SJ through the physical task (single task) may be different from that of the physical task included in the dual task.

The cognitive function evaluation system 100 according to the present embodiment will be further described with reference to FIG. 1. As depicted in FIG. 1, the cognitive function evaluation system 100 further includes a motion detector 20, an answer detector 30, and an evaluator 40.

The motion detector 20 captures images of a subject SJ performing a predetermined task to generate frames representing three-dimensional coordinates of all joints of the subject SJ whose images have been captured. Here, the frames are a series of frames generated in time order. Specifically, the motion detector 20 includes an image-capturing section 21 and a motion-capturing section 22.

The image-capturing section 21 captures images of a subject SJ. Specifically, the image-capturing section 21 captures the images of a subject SJ performing the dual task. In the present embodiment, the images of the subject SJ performing the physical task (single task) are further captured. Examples of the image-capturing section 21 include a CCD image sensor, a CMOS image sensor, and a range sensor. The image-capturing section 21 is placed in front of the subject SJ, for example. All the joints of the subject SJ can be captured by placing the image-capturing section 21 in front of the subject SJ.

The motion-capturing section 22 transforms the motion of each part of the subject SJ into vector data to generate motion capture data that reflects the motion of each part of the subject SJ (motion of the subject SJ). Specifically, the motion capture data contains continuous frames representing a human skeleton model that moves according to the motion of the subject SJ whose images have been captured by the image-capturing section 21. The human skeleton model represents a skeleton model of the subject SJ by a tree structure in which adjacent joints are linked based on the human body structure. The human skeleton model (frames) represents the three-dimensional coordinates of all the joints of the subject SJ whose images have been captured. In other words, the human skeleton model is a three-dimensional human skeleton model.

The motion-capturing section 22 includes, for example, a processor and storage. Examples of the processor include a central processing unit (CPU) and a micro processing unit (MPU). The storage stores a computer program to be executed by the processor. The computer program includes a computer program for generating motion capture data from the output of the image-capturing section 21. Examples of the storage include semiconductor memory such as read-only memory (ROM) and random-access memory (RAM).

The answer detector 30 detects answers to questions on a predetermined cognitive examination by a subject SJ performing a predetermined task. Specifically, the answer detector 30 detects the answers from the subject SJ performing the dual task. In the present embodiment, answers from the subject SJ performing the cognitive task (single task) are further detected.

In the present embodiment, the answer detector 30 includes a left-hand answer switch and a right-hand answer switch. By pushing the right-hand answer switch or the left-hand answer switch, the subject SJ is to answer the questions on the cognitive examination presented by the task-presenting section 10.

Note that the left-hand answer switch may be held in the left hand of the subject SJ or may be fixed to a handrail installed on the left side of the subject SJ. Similarly, the right-hand answer switch may be held in the right hand of the subject SJ or may be fixed to a handrail installed on the right side of the subject SJ.

The evaluator 40 outputs motion features based on the frames acquired from the motion detector 20. The motion features represent a feature of a spatial positional relationship and a feature of temporal variations, of all the joints of the subject SJ whose images have captured. The evaluator 40 then evaluates a cognitive function of the subject SJ based on the motion features and the answers detected by the answer detector 30.

In the present embodiment, the evaluator 40 determines a cognitive function score of the subject SJ. The evaluator 40 also classifies the subject SJ into a class in which the cognitive function score is less than or equal to a threshold or a class in which the cognitive function score is greater than the threshold. Specifically, according to the threshold that is set in advance, the subject SJ is classified into a class of dementia or a class of mild cognitive impairment and non-dementia, or into a class of dementia and mild cognitive impairment or a class of non-dementia.

For example, the cognitive function score is an MMSE score, and the threshold is 23 points. In this example, the evaluator 40 classifies a subject SJ into a class in which the MMSE score is less than or equal to 23 points or a class in which the MMSE score is greater than 23 points. In addition, the cognitive function score is an MMSE score, and the threshold is 27 points. In this case, the evaluator 40 classifies the subject SJ into a class in which the MMSE score is less than or equal to 27 points or a class in which the MMSE score is greater than 27 points.

An example of a task that the task-presenting section 10 presents to a subject SJ will be described with reference to FIGS. 2A to 2D. FIGS. 2A to 2D illustrate the example of the task that the task-presenting section 10 presents to the subject SJ. In the example of FIGS. 2A to 2D, a behavior to be performed by the subject SJ is "stepping on the spot", and questions on a cognitive examination to be presented to the subject SJ includes "calculation problems".

As depicted in FIG. 2A, the task-presenting section 10 first displays a first notification screen 11 notifying a subject SJ of the start of the task. As depicted in FIG. 2B, the task-presenting section 10 then requires the subject SJ to perform a cognitive task (single task). Specifically, the task-presenting section 10 displays a question-presenting screen 12a that presents a calculation problem (a question on the cognitive examination). For example, the task-presenting section 10 presents a subtraction problem on the display as a calculation problem.

Note that although the calculation problem in the example of FIG. 2B is the subtraction problem, the calculation problem is not limited to any subtraction problems. The calculation problem may be an addition problem. Alternatively, the calculation problem may include a subtraction problem and an addition problem.

The task-presenting section 10 finishes presenting the calculation problem (question on cognitive examination) at a predetermined timing. Specifically, the question-presenting screen 12a is removed from the display. After the question-presenting screen 12a is removed from the display, the task-presenting section 10 presents an answer-candidate-presenting screen 12b including two answer candidates. The subject SJ presses one of the two answer switches to select an answer.

The subject SJ selects an answer, and the task-presenting section 10 then presents the next problem that is a calculation problem (question on cognitive examination) different from the calculation problem (question on cognitive examination) answered this time. Thereafter, calculation questions (questions on cognitive examination) to be answered by the subject SJ are repeatedly presented until a predetermined cognitive task performance time has elapsed.

When the predetermined cognitive task performance time has elapsed, the task-presenting section 10 presents a physical task (single task) to the subject SJ as depicted in FIG. 2C. Specifically, the task-presenting section 10 presents a behavior presentation screen 13 that requires the subject SJ to perform a behavior (stepping).

In the present embodiment, the behavior required for the subject SJ in the dual task is the same as the behavior required for the subject SJ in the physical task (single task). In addition, the questions on the cognitive examination required for the subject SJ in the dual task are the same as the questions on the cognitive examination required for the subject SJ in the cognitive task (single task). Therefore, when a predetermined physical task performance time has elapsed, the task-presenting section 10 repeatedly presents calculation problems (questions on cognitive examination) to be answered by the subject SJ until a predetermined dual task performance time has elapsed. When the predetermined dual task performance time has elapsed, the task-presenting section 10 presents a second notification screen 14 on the display to notify the subject SJ of the end of the task as depicted in FIG. 2D.

Note that the questions on cognitive examination presented by the task-presenting section 10 are not limited to any calculation problems. For example, the questions on cognitive examination may be location memory problems or rock paper scissors problems.

FIG. 3 is a diagram depicting another example of questions on a cognitive examination that a task-presenting section 10 presents to a subject SJ. The questions on the cognitive examination in the example of FIG. 3 are "location memory problems". As depicted in FIG. 3, when the questions on the cognitive examination are the location memory problems, the task-presenting section 10 presents a question-presenting screen 12a in which a figure is placed in one of four areas in which a figure can be placed.

The task-presenting section 10 then removes the question-presenting screen 12a from the display. The task-presenting section 10 subsequently presents an answer-candidate-presenting screen 12b in which a figure is placed in one of four areas in which a figure can be placed. The answer-candidate-presenting screen 12b includes "Yes" and "No" as two answer candidates along with a question sentence. The question sentence describes a question that can be answered with "Yes" or "No". Here, the subject SJ is asked whether or not the positions where respective figures are placed are the same between the question-presenting screen 12a and the answer-candidate-presenting screen 12b.

For example, the subject SJ determines that the positions where the figures are placed are the same between the question-presenting screen 12a and the answer-candidate-presenting screen 12b. In this case, the subject SJ presses the left-hand answer switch to select "Yes". Alternatively, the subject SJ determines that the positions where the figures are placed are different between the question-presenting screen 12a and the answer-candidate-presenting screen 12b. In this case, the subject SJ presses the right-hand answer switch to select "No".

Rock paper scissors problems will now be described. Here, questions on a cognitive examination required for a subject SJ through a cognitive task are "rock paper scissors problems". In this case, a task-presenting section 10 presents one of "rock", "scissors", and "paper" on a question-presenting screen 12a. The task-presenting section 10 then displays one of "rock", "scissors", and "paper"; a question sentence; and "yes" and "no" on an answer-candidate-presenting screen 12b. For example, in the answer-candidate-presenting screen 12b, the subject SJ is asked whether the finger pose displayed on the answer-candidate-presenting screen 12b can beat the finger pose displayed on the question-presenting screen 12a.

For example, the subject SJ determines that the finger pose displayed on the answer-candidate-presenting screen 12b can beat the finger pose displayed on the question-presenting screen 12a. In this case, the subject SJ presses the left-hand answer switch to select "Yes". Alternatively, the subject SJ determines that the finger pose displayed on the answer-candidate-presenting screen 12b loses to the finger pose displayed on the question-presenting screen 12a. In this case, the subject SJ presses the right-hand answer switch to select "No".

A cognitive function evaluation system 100 according to the present embodiment will be described with reference to FIG. 4. FIG. 4 is a block diagram depicting a configuration of the cognitive function evaluation system 100 according to the present embodiment. Specifically, FIG. 4 depicts the configuration of an evaluator 40. As depicted in FIG. 4, the cognitive function evaluation system 100 further includes an identification-information-acquiring section 50 and an output section 60. The evaluator 40 includes storage 41 and a processor 42.

Subjects SJ utilizing the cognitive function evaluation system 100 are assigned their respective unique identification information in advance. The identification-information-acquiring section 50 acquires the identification information assigned to a subject SJ. Examples of the identification-information-acquiring section 50 include a card reader, a keyboard, and a touch panel. If the identification-information-acquiring section 50 is the card reader, the subject SJ causes the card reader to read the identification information carried on a card of the subject. If the identification-information-acquiring section 50 is the keyboard or the touch panel, the subject SJ operates the keyboard or the touch panel to enter the identification information assigned to the subject SJ.

The output section 60 outputs evaluation results of a cognitive function of the subject SJ. In the present embodiment, the evaluation results include a cognitive function classification result and a cognitive function score.

The classification result indicates a class into which the cognitive function of the subject SJ is classified. Specifically, classes into which respective cognitive functions of subjects SJ will be classified include a class of dementia and a class of mild cognitive impairment and non-dementia. Alternatively, the classes into which the cognitive functions of subjects SJ will be classified include a class of dementia and mild cognitive impairment and a class of non-dementia. For example, the cognitive function of a subject SJ is classified into a class in which an MMSE score is less than or equal to 23 points or a class in which the MMSE score is greater than 23 points. Alternatively, the cognitive function of a subject SJ is classified into a class in which an MMSE score is less than or equal to 27 points or a class in which the MMSE score is greater than 27 points. The output section 60 is, for example a printer. The printer prints and outputs the evaluation results of the cognitive function of a subject SJ on paper.

Note that the output section 60 is not limited to any printers. The output section 60 may be, for example a communication section. For example, the communication section may send an email indicating the evaluation results of the cognitive function of a subject SJ to an email address registered in advance by the subject SJ.

The storage 41 stores a computer program and various data. The storage 41 includes, for example a semiconductor memory. Examples of the semiconductor memory include RAM and ROM. The semiconductor memory may further include video RAM (VRAM). Examples of the storage 41 may further include a hard disk drive (HDD) and a solid-state drive (SSD).

In the present embodiment, the storage 41 stores evaluation results of a cognitive function of each subject SJ in association with the identification information assigned to a corresponding subject SJ. In other words, the storage 41 stores the history of evaluation results of the cognitive function of each subject SJ. The storage 41 also stores a trained model TM. The trained model TM is a computer program for evaluating a cognitive function of a subject SJ based on frames acquired from a motion detector 20 and answers detected by an answer detector 30.

The processor 42 executes the computer program stored in the storage 41 to perform various processes such as numerical calculation, information processing, and device control. Examples of the processor 42 may include a CPU and an MPU. The processor 42 may further include a graphics processing unit (GPU) or a neural network processing unit (NPU). Alternatively, the processor 42 may include a quantum computer.

Specifically, when the identification-information-acquiring section 50 acquires identification information, the processor 42 then causes a task-presenting section 10 to present various screens as described with reference to FIGS. 2A to 2D and 3. The processor 42 also enters frames acquired from the motion detector 20 into the trained model TM. The processor 42 further extracts features from answers detected by the answer detector 30 and then enters the features extracted from the answers into the trained model TM. In the present embodiment, the frames acquired from the motion detector 20 and the features of the answers by a subject SJ are entered into the trained model TM.

More specifically, from frames acquired from the motion detector 20, the processor 42 extracts continuous frames acquired during the performance of a physical task (single task) and continuous frames acquired during the performance of a dual task. The processor 42 then enters the frames into the trained model TM. The number of frames in the physical task (single task) is, for example 160 frames. The number of frames in the dual task is, for example 260 frames.

Based on answers detected by the answer detector 30, the processor 42 finds out features of the answers, which include an answer speed and a correct answer rate. The answer speed is expressed by the number of times a subject SJ has answered per unit time. The unit time is, for example 1 second. The correct answer rate is expressed as a ratio of the number of correct answers to the number of answers. The number of answers is the number of times the subject SJ has answered. The number of correct answers is the number of answers that the subject has correctly responded.

Here, the answer speed is calculated with respect to the total time of the cognitive task performance time and the dual task performance time. That is, the answer speed is calculated by dividing a total value by a total time. The total value is a total value of the number of times the subject SJ has answered during the performance of the cognitive task (single task) and the number of times the subject SJ has answered during the performance of the dual task. The total time is a total time of the cognitive task performance time and the dual task performance time. The correct answer rate is also expressed as a ratio of a first total value to a second total value. The first total value is a total value of the number of times the subject SJ has correctly answered questions presented during the performance of the cognitive task (single task) and the number of times the subject SJ has correctly answered questions presented during the performance of the dual task. The second total value is a total value of the number of times the subject SJ has answered during the performance of the cognitive task (single task) and the number of times the subject SJ has answered during the performance of the dual task.

Note that the features of the answers are not limited to any answer speeds and correct answer rates. For example, the features of the answers may be only one of the answer speed and the correct answer rate. Alternatively, the features of the answers may include at least one of the number of answers, the number of correct answers, an average answer time interval, and a standard deviation of data on answer time intervals, instead of or in addition to the answer speed and the correct answer rate. Here, each answer time interval is a time interval taken from when the task-presenting section 10 presents an answer-candidate-presenting screen 12b as described with reference to FIGS. 2B and 3 to when the subject SJ presses an answer switch.

The processor 42 acquires evaluation results of the cognitive function of a subject SJ based on the output of the trained model TM. The processor 42 acquires the evaluation results of the cognitive function of the subject SJ, and then causes the output section 60 to output the evaluation results of the cognitive function of the subject SJ. Note that the processor 42 may cause the output section 60 to output the history of evaluation results of the cognitive function of the subject SJ. The history of evaluation results that the output section 60 outputs may be expressed in a table format or a graph format.

The processor 42 evaluates the cognitive function of a subject SJ and then causes the storage 41 to store the evaluation results of the cognitive function of the subject SJ in association with a corresponding identification information. As a result, the storage 41 stores the history of evaluation results of the cognitive function of each subject SJ in association with a corresponding identification information.

An example of a task to be performed by a subject SJ will now be described. In the present embodiment, the subject SJ repeats a task set TA three times continuously. The task set TA requires the subject SJ to perform a cognitive task, a physical task, and a dual task in this order. The task set TA to be performed for the first time is also hereinafter referred to as a "first task set TA1". Similarly, the task sets TA and TA to be performed for the second and third times are also hereinafter referred to as "second and third task sets TA2 and TA3", respectively.

Furthermore, frames acquired from a performance of one task set TA by a subject SJ are also hereinafter referred to as a "frame group". In addition, respective frame groups acquired from respective performances of the first to third task sets TA1 to TA3 by the subject SJ are also hereinafter referred to as "first to third frame groups". Similarly, features-of-answers acquired from a performance of one task set TA by a subject SJ are also hereinafter referred to as "answer feature data". In addition, respective features-of-answers acquired from respective performances of the first to third task sets TA1 to TA3 by the subject SJ are also hereinafter referred to as "first to third answer feature data".

A trained model TM will now be described with reference to FIG. 5. FIG. 5 illustrates a neural network NW built by the trained model TM. The neural network NW is, for example a neural network that performs a process of deep learning.

As depicted in FIG. 5, the neural network NW in the present embodiment includes motion-feature-extracting sections 2, transforming sections 3, a combining section 4, a convolving section 5, a combining section 6, and a score-determining section 7.

A (Each) motion-feature-extracting section 2 is supplied with a frame group. The motion-feature-extracting section 2 captures a human skeleton model as a graph structure. Specifically, the motion-feature-extracting section 2 captures two graph structures from the frame group. That is, the motion-feature-extracting section 2 generates respective spatial graphs for the frames. Each spatial graph indicates respective spatial (three-dimensional) positional relationships of all joints of a subject SJ whose images have been captured by an image-capturing section 21. The motion-feature-extracting section 2 also generates time graphs across the frames. Each time graph represents respective variations (i.e., temporal changes in joints) in an identical joint between each adjacent frames. The respective spatial graphs and the time graphs are then convolved, so that motion features as described with reference to FIG. 1 are extracted. That is, the features of the spatial positional relationships of all the joints are extracted by convolving the spatial graphs. The features of respective temporal variations of all the joints are extracted by convolving the time graphs. The motion-feature-extracting section 2 includes, for example, a graph convolutional neural network such as a spatio-temporal graph convolutional neural network (ST-GCN).

For example, assume that a behavior to be required for a subject SJ is stepping (stepping on the spot). In this case, the motion-feature-extracting section 2 extracts a spatio-temporal feature (motion feature) of the stepping by the subject SJ from the spatial (3D) positional relationship and the respective temporal variations, of all joints included in the 3D human skeleton model.

In the present embodiment, a task set TA is performed a plurality of times continuously, so that task sets TA are performed. The task sets TA are separately entered into the motion-feature-extracting sections 2. Specifically, the motion-feature-extracting sections 2 include first to third motion-feature-extracting sections 2a to 2c. The first to third frame groups are entered into the first to third motion-feature-extracting sections 2a to 2c, respectively.

A (Each) transforming section 3 transforms a motion feature (spatio-temporal feature of subject's, SJ, motion) generated by a motion-feature-extracting section 2 into a scalar value. The transforming section 3 includes, for example a fully connected layer (FC). The transforming sections 3 in the present embodiment include first to third transforming sections 3a to 3c. The first to third transforming sections 3a to 3c are supplied with respective outputs of the first to third motion-feature-extracting sections 2a to 2c.

The combining section 4 combines respective scalar values from the transforming sections 3. In the present embodiment, the combining section 4 combines respective outputs of the first to third motion-feature-extracting sections 2a to 2c. As a result, the combining section 4 outputs a scalar value acquired by combining respective motion features extracted from the first to third frame groups. That is, the output of the combining section 4 reflects the respective motion features extracted from the first to third frame groups. The combining section 4 includes, for example a fully connected layer (FC).

The convolving section 5 convolves pieces of answer feature data. In the present embodiment, the convolving section 5 convolves first answer feature data to third answer feature data. The convolving section 5 includes, for example a convolutional neural network (CNN).

The combining section 6 outputs scalar values s based on answer feature data and a motion feature. Specifically, the combining section 6 combines the output (a scalar value reflecting motion features) of the combining section 4 and answer feature data convolved by the convolving section 5, and then outputs the scalar values s. Here, the scalar values s include a scalar value s1 for positive and a scalar value s2 for negative. The scalar value s1 for positive is a value corresponding to a probability that a subject SJ is identified as true positive. The scalar value s2 for negative is a value corresponding to a probability that a subject SJ is identified as true negative. Here, positive indicates that the cognitive function score is below a threshold (e.g., MMSE score is less than or equal to 23 or 27 points). Negative indicates that the cognitive function score is greater than the threshold (e.g., MMSE score is greater than 23 or 27 points). The combining section 6 includes, for example a fully connected layer (FC).

A processor 42 as described with reference to FIG. 4 classifies the cognitive function of a subject SJ based on the scalar values s acquired from the neural network NW (trained model TM). For example, the processor 42 compares the scalar value s1 for positive and the scalar value s2 for negative. The processor 42 then determines that the subject SJ is positive if the scalar value s1 for positive is greater than the scalar value s2 for negative. On the other hand, the processor 42 determines that the subject SJ is negative if the scalar value s2 for negative is greater than the scalar value s1 for positive.

The score-determining section 7 determines a cognitive function score. Specifically, the score-determining section 7 is supplied with the scalar values s. The score-determining section 7 determines the cognitive function score based on the scalar values s. The processor 42 as described with reference to FIG. 4 acquires the cognitive function score of the subject SJ from the output of the score-determining section 7. The score-determining section 7 includes, for example a fully connected layer (FC).

The approach by the embodiment described above with reference to FIGS. 1 to 5 enables evaluation of the cognitive function of a subject SJ based on respective features of the spatial positional relationship and temporal variations, of all joints of the subject SJ whose images have been captured. It is therefore possible to more accurately evaluate the cognitive function of the subject SJ, compared to a system that evaluates the cognitive function of a subject SJ based on limited features.

A learning method according to the present embodiment will be described with reference to FIGS. 6 to 10. The learning method according to the present embodiment includes determining a value of each parameter (parameter values) included in a trained model TM. FIG. 6 is a diagram depicting a model-generating system 200 that generates the trained model TM.

As depicted in FIG. 6, the model-generating system 200 includes a task-presenting section 10, a motion detector 20, an answer detector 30, an evaluator 40, an identification-information-acquiring section 50, and a data-collecting section 70.

The task-presenting section 10 presents a task to a subject SJ as described with reference to FIGS. 1 to 5. The motion detector 20 outputs frames as described with reference to FIGS. 1 to 5. The identification-information-acquiring section 50 acquires the identification information on the subject SJ as described with reference to FIGS. 1 to 5.

The data-collecting section 70 collects data acquired by the subject SJ performing first to third task sets TA1 to TA3 as described with reference to FIGS. 1 to 5. The data-collecting section 70 then generates training data. Specifically, the data-collecting section 70 extracts and collects first to third frame groups from the output of the motion detector 20. The data-collecting section 70 also extracts and collects features of answers from the output of the answer detector 30. Note that the data collected by the data-collecting section 70 may include pieces of data acquired from the same subject SJ.

A model-generating system 200 will be described with reference to FIG. 7. FIG. 7 is a block diagram depicting a partial configuration of the model-generating system 200. Specifically, FIG. 7 depicts the configuration of a data-collecting section 70. As depicted in FIG. 7, the data-collecting section 70 includes an input section 71, storage 72, and a processor 73. The data-collecting section 70 is, for example a server.

The input section 71 allows an operator to operate. The operator operates the input section 71, thereby causing the input section 71 to enter various information into the processor 73. Examples of the input section 71 includes input devices such as a keyboard, a mouse, and a touch panel. For example, the operator operates the input section 71 to enter label data (teacher data) to be used in supervised learning or semi-supervised learning. In the present embodiment, the operator enters a cognitive function score of a subject SJ as label data (teacher data) in order to create a trained model TM for determining a cognitive function score.

Further, the operator enters label data indicating positive in association with each subject SJ whose cognitive function score is less than or equal to a threshold, while entering label data indicating negative in association with each subject SJ whose cognitive function score is greater than the threshold. For example, assume that a trained model TM is generated that classifies subjects SJ into a class of dementia or a class of mild cognitive impairment and non-dementia. In this case, label data indicating positive is entered in association with each subject SJ whose MMSE score is less than or equal to 23 points, while label data indicating negative is entered in association with each subject SJ whose MMSE score is greater than 23 points. Assume that a trained model TM is generated that classifies subjects SJ into a class of dementia and mild cognitive impairment or a class of non-dementia. In this case, label data indicating positive is entered in association with each subject SJ whose MMSE score is less than or equal to 27 points, while label data indicating negative is entered in association with each subject SJ whose MMSE score is greater than 27 points. Note that such a cognitive function score may be acquired by having subjects SJ take a written test (e.g., MMSE written test) in advance.

The storage 72 stores a computer program and various data. The storage 72 includes, for example a semiconductor memory. Examples of the semiconductor memory include RAM and ROM. The semiconductor memory may further include VRAM. Examples of the storage 72 may further include an HDD and an SSD.

In the present embodiment, the storage 41 stores task-related data of each subject SJ in association with identification information assigned to a corresponding subject SJ. The task-related data contains first to third frame groups and first to third answer feature data as described with reference to FIGS. 1 to 5. The task-related data further contains label data (teacher data) entered from the input section 71. The storage 41 may store a database of task-related data, for example. Note that pieces of task-related data acquired from the same subject SJ are managed as different data.

The processor 73 executes the computer program stored in the storage 72 to perform various processes such as numerical calculation, information processing, and device control. The processor 73 may include, for example, a CPU or an MPU.

Specifically, the processor 73 causes the storage 72 to store the label data (teacher data) entered from the input section 71 in association with the identification information of a corresponding subject SJ. An identification-information-acquiring section 50 acquires identification information, and the processor 73 then causes a task-presenting section 10 to present various screens as described with reference to FIGS. 2 and 3. The processor 73 also acquires first to third frame groups from the output of a motion detector 20 and causes the storage 72 to store the groups in association with the identification information of the subject SJ. The processor 73 further extracts first to third answer feature data from the output of an answer detector 30 and causes the storage 72 to store the first to third answer feature data in association with the identification information of the subject SJ.

The processor 73 generates training data based on the task-related data stored in the storage 72. The processor 73 then outputs the training data to an evaluator 40. In the present embodiment, the evaluator 40 performs mini-batch learning. The processor 73 accordingly generates data for mini-batch learning and outputs the data generated to the evaluator 40.

A model-generating system 200 will be described with reference to FIG. 8. FIG. 8 is a block diagram depicting a configuration of an evaluator 40 during learning. As depicted in FIG. 8, the evaluator 40 further includes an input section 43.

The input section 43 allows an operator to operate. The operator operates the input section 43, thereby causing the input section 43 to enter various information into a processor 42. Examples of the input section 43 include input devices such as a keyboard, a mouse, and a touch panel. For example, the operator operates the input section 43 to enter a loss function, a learning rate, and a dropout value. For example, the learning rate is set to 0.1. The dropout value is set to 0.8.

During learning, the storage 41 stores a training program TP. The training program TP is a program for executing an algorithm that is used to find a certain rule from training data to generate a model (trained model TM) expressing the rule.

The processor 42 provides the loss function, the learning rate, and the dropout value to the training program TP. The processor 42 subsequently uses training data (mini-batch training data) acquired from a data-collecting section 70 to execute the training program TP. As a result, a neural network NW built by the training program TP is trained, and a trained model TM is generated. Specifically, the trained model TM is generated by determining respective values of parameters (parameter values) included in the neural network NW to values that minimizes the loss function.

Here, the loss function will be described. In the present embodiment, the processor 42 determines the parameter values using the loss function. The loss function optimizes the sum (P1+P2) of sensitivity P1 and specificity P2. The sensitivity P1 indicates a rate at which subjects SJ can be identified as true positive. The specificity P2 indicates a rate at which subjects SJ can be identified as true negative.

Specifically, the processor 42 searches for a minimum value of the loss function through a gradient method. That is, the processor 42 searches for the minimum value of the loss function by partially differentiating the loss function. In the present embodiment, the processor 42 works out a gradient based on Equations (1) to (4) below. Equation (1) is a loss function. Equation (2) is an equation acquired by partially differentiating Equation (1). Equation (3) represents "weight".

[Math 1]

$$\tilde{L} = -w(N_P, N_N)\left(\frac{1}{N_P}\sum_{i \in S_P}\log(f(s_i)) + \frac{1}{N_N}\sum_{i \in S_N}\log(1-f(s_i))\right) \quad \cdots (1)$$

[Math 2]

$$\frac{\partial \tilde{L}}{\partial s_i} = \begin{cases} \frac{w(N_P, N_N)}{N_P}\left(1 - f(s_i)\right) & (i \in S_P) \\ -\frac{w(N_P, N_N)}{N_N} f(s_i) & (i \in S_N) \end{cases} \qquad \cdot \cdot \cdot (2)$$

[Math 3]

$$w(N_P, N_N) = \frac{2 N_P N_N}{(N_P + N_N)} \qquad \cdot \cdot \cdot (3)$$

[Math 4]

$$f(s) = \frac{1}{1 + exp(-s)}, \qquad \cdot \cdot \cdot (4)$$

In Equations (1) to (3), $N_P$ indicates the number of positive samples included in the mini-batch, and $N_N$ indicates the number of negative samples included in the mini-batch. Weight $w(N_P, N_N)$ therefore indicates a harmonic mean of the number of positive samples and the number of negative samples, included in the mini-batch. $S_P$ and $S_N$ indicate positive and negative indices included in the mini-batch, respectively. As depicted in Equation (4), $f(s_i)$ is a sigmoid function with scalar values s as variables. Note that $s_i$ indicates the i-th sample. In Equations (1) and (2), "$f(s_i)$" indicates a probability that the i-th sample is positive, and "$1-f(s_i)$" indicates a probability that the i-th sample is negative. Here, "$f(s_i)$" is assigned a scalar value s1 for positive, and "$1-f(s_i)$" is assigned a scalar value s2 for negative.

Note that Equation (1) is a logarithmic version of the loss function L depicted in Equation (5) below, with weight $w(N_P, N_N)$.

[Math 5]

$$L = -\left(\tilde{P}_{sensitivity} + \tilde{P}_{specificity}\right)$$
$$= -\left(\frac{1}{N_P}\sum_{i \in S_P} f(s_i) + \frac{1}{N_N}\sum_{i \in S_N}\left(1 - f(s_i)\right)\right) \qquad \cdot \cdot \cdot (5)$$

Equation (5) includes Equations (6) and (7) below. Equation (6) is an equation for mitigating the sensitivity P1 using a sigmoid function with scalar values s as variables. Equation (7) is an equation for mitigating the specificity P2 using a sigmoid function with scalar values s as variables.

[Math 6]

$$\tilde{P}_{sensitivity} = \frac{1}{N_P}\sum_{i \in S_P} f(s_i) \qquad \cdot \cdot \cdot (6)$$

[Math 7]

$$\tilde{P}_{specificity} = \frac{1}{N_N}\sum_{i \in S_N}\left(1 - f(s_i)\right) \qquad \cdot \cdot \cdot (7)$$

Equation (1) described above is therefore a loss function including the sum (P1+P2) of sensitivity P1 and specificity P2. It is possible to determine parameter values that optimize the sum (P1+P2) of sensitivity P1 and specificity P2 by searching for the minimum value of the loss function depicted in Equation (1) based on Equation (2). The approach by Equation (1) enables performance of learning using a backpropagation learning method because the gradient does not disappear. In addition, although there may be an imbalance between the number of positive and negative samples, the influence can be reduced by introducing the weight depicted in Equation (3).

A training program TP will now be described with reference to FIGS. 9 and 10. FIG. 9 illustrates a neural network NW built by the training program TP. In other words, FIG. 9 depicts the neural network NW during learning. FIG. 10 is a diagram depicting a flow chart of a learning method according to the present embodiment.

The learning method according to the present embodiment includes a method of determining parameter values of the neural network NW by learning from frame groups. The method of determining parameter values of the neural network NW includes a method of determining parameter values of motion-feature-extracting sections 2. FIG. 10 depicts a method for determining the parameter values of the motion-feature-extracting sections 2 (first to third motion-feature-extracting sections 2a to 2c). As depicted in FIG. 10, the method for determining the parameter values of the first to third motion-feature-extracting sections 2a to 2c includes Steps S1 to S5.

In the present embodiment, a processor 42 first determines parameter values of the first motion-feature-extracting section 2a (Step S1), as depicted in FIGS. 9 and 10. Specifically, the processor 42 enters first frame groups into the first motion-feature-extracting section 2a to cause the first motion-feature-extracting section 2a to learn from the first frame groups. For example, the processor 42 performs learning using a backpropagation learning method. As a result, the parameter values of the first motion-feature-extracting section 2a are determined.

The processor 42 determines the parameter values of the first motion-feature-extracting section 2a, and then sets the parameter values of the first motion-feature-extracting section 2a to initial values of the parameter values of the second motion-feature-extracting section 2b (Step S2).

The processor 42 sets the initial values of the parameter values of the second motion-feature-extracting section 2b, and then determines the parameter values of the second motion-feature-extracting section 2b (Step S3). Specifically, the processor 42 enters second frame groups into the second motion-feature-extracting section 2b to cause the second motion-feature-extracting section 2b to learn from the second frame groups. That is, the second motion-feature-extracting section 2b uses the parameter values of the first motion-feature-extracting section 2a as its own initial values and then learns from the second frame groups. For example, the processor 42 performs learning using a backpropagation learning method. As a result, the parameter values of the second motion-feature-extracting section 2b are determined.

The processor 42 determines the parameter values of the second motion-feature-extracting section 2b, and then sets the parameter values of the second motion-feature-extracting section 2b to initial values of the parameter values of the third motion-feature-extracting section 2c (Step S4).

The processor 42 sets the initial values of the parameter values of the third motion-feature-extracting section 2c, and then determines the parameter values of the third motion-feature-extracting section 2c (Step S5). Specifically, the processor 42 enters third frame groups into the third motion-feature-extracting section 2c to cause the third motion-feature-extracting section 2c to learn from the third frame groups. That is, the third motion-feature-extracting section 2c uses the parameter values of the second motion-feature-extracting section 2b as its own initial values and then learns from the third frame groups. For example, the processor 42 performs learning using a backpropagation learning method. As a result, the parameter values of the third motion-feature-extracting section 2c are determined.

In the present embodiment, in order to determine parameter values of a specific motion-feature-extracting section 2, previously determined parameter values of another motion-feature-extracting section 2 are set as initial values of parameter values of the specific motion-feature-extracting section 2. This approach makes it possible to efficiently determine the parameter values.

Note that although in the present embodiment, subjects SJ are required to perform a task set TA three times continuously, the cognitive function evaluation system 100 may be a system that requires subjects SJ to perform a task set TA once, twice continuously, or four times or more continuously. The number of motion-feature-extracting sections 2 and transforming sections 3 may be adjusted according to the number of times the task set TA is performed continuously.

FIG. 11 is a diagram depicting another example of a neural network NW built by a trained model TM. Specifically, the neural network NW depicted in FIG. 11 is included in a cognitive function evaluation system 100 that requires subjects SJ to perform a task set TA once. In FIG. 11, subjects SJ are required to perform the task set TA once. In this case, the system includes one motion-feature-extracting section 2 and one transforming section 3. In this configuration that requires subjects SJ to perform the task set TA once, a convolving section 5 as described with reference to FIG. 5 may or may not be omitted.

In the present embodiment, label data indicating positive is entered in association with each subject SJ whose cognitive function score is less or equal to a threshold, while label data indicating negative is entered in association with each subject SJ whose cognitive function score is greater than the threshold. However, label data indicating positive and label data indicating negative may be entered based on definitive diagnoses made by doctors.

Assumed that a trained model TM is generated that classifies a cognitive function of each subject SJ into a class of dementia or a class of cognitive impairment and non-dementia. In this case, label data indicating positive is entered in association with each subject SJ who has been given a definitive diagnosis of dementia, while label data indicating negative is entered in association with each subject SJ who has been given a definitive diagnosis of mild cognitive impairment or non-dementia. Thus, a cognitive function of each subject SJ can be classified through definitive diagnoses into a class of dementia or a class of mild cognitive impairment and non-dementia without using a cognitive function score and thresholds for the cognitive function score. The same holds true when generating a trained model TM that classifies a cognitive function of each subject SJ into a class of dementia and mild cognitive impairment or a class of non-dementia. In this case, since no cognitive function score is used, the cognitive function evaluation system 100 doesn't need to determine a cognitive function score of each subject SJ. A score-determining section 7 as described with reference to FIGS. 5 and 9 is therefore omitted.

In the present embodiment, parameter values are determined using the loss function described with reference to Equations (1) to (7). However, the loss function is not limited to loss functions described with reference to Equations (1) to (7). The loss function may be any of known loss functions which include mean square error (MSE), mean absolute error (MAE), root-mean-square error (RMSE), mean square log error (MSLE), Huber loss, Poisson loss, hinge loss, and Kullback-Leibler divergence (KLD).

[Second Embodiment]

A second embodiment 2 of the present invention will now be described with reference to FIG. 12. However, matters that are different from the first embodiment will be described, and descriptions of matters that are the same as the first embodiment will be omitted. The second embodiment differs from the first embodiment in that an evaluator 40 classifies respective cognitive functions of subjects SJ into a class of dementia, a class of mild cognitive impairment, or a class of non-dementia. That is, in the second embodiment, the evaluator 40 classifies the cognitive functions of the subjects SJ into the three classes.

In the present embodiment, the cognitive functions of the subjects SJ each are classified into the class of dementia, the class of mild cognitive impairment, or the class of non-dementia. In this case, during learning, respective definitive diagnoses by doctors are entered into a processor 73 (FIG. 7) as respective pieces of label data (teacher data). Specifically, an operator enters, through an input section 71 (FIG. 7), label data indicating dementia, label data indicating mild cognitive impairment, and label data indicating non-dementia.

In the case where respective cognitive functions of subjects SJ each are classified into the class of dementia, the class of mild cognitive impairment, or the class of non-dementia, a loss function to be used is, for example, any of known loss functions which include cross entropy loss, triplet loss, and center loss. Note that mini-batch learning can be performed even when respective cognitive functions of subjects SJ are classified into the three classes. The processor 73 (FIG. 7) may therefore generate data for mini-batch learning to be output to the evaluator 40, and the evaluator 40 (FIG. 6) may perform the mini-batch learning, like the first embodiment.

FIG. 12 is a diagram depicting a neural network NW included in a cognitive function evaluation system 100 according to the present embodiment. The neural network NW (trained model TM) included in the cognitive function evaluation system 100 according to the present embodiment differs from the neural network NW depicted in FIG. 5 in that a combining section 6a is included as depicted in FIG. 12. The neural network NW included in the cognitive function evaluation system 100 according to the present embodiment also differs from the neural network NW depicted in FIG. 5 in that transforming sections 3 (first to third transforming sections 3a to 3c) and a score-determining section 7 are not included.

In the present embodiment, a combining section 4 is supplied with motion features extracted by each of first to third motion-feature-extracting sections 2a to 2c. The combining section 4 combines respective motion features supplied from the first to third motion-feature-extracting sections 2a to 2c. The output of the combining section 4 therefore reflects the respective motion features extracted from first to third frame groups.

The combining section 6a combines the output of the combining section 4 and answer feature data convolved by a convolving section 5. The combining section 6a then outputs a probability that a subject SJ is dementia, a probability that the subject SJ is mild cognitive impairment, and a probability that the subject SJ is non-dementia. The combining section 6a includes, for example a fully connected layer (FC).

In the present embodiment, a processor 42 as depicted in FIG. 4 classifies the cognitive function of a subject SJ into a class of dementia, a class of mild cognitive impairment, or a class of non-dementia based on the output of the neural network NW (combining section 6a). For example, assume that the probability that the subject SJ is dementia is maximum within the probability that the subject SJ is dementia, the probability that the subject SJ is mild cognitive impairment, and the probability that the subject SJ is non-dementia. In this case, the processor 42 as depicted in FIG. 4 classifies the cognitive function of the subject SJ into the class of dementia.

The second embodiment has been described above with reference to FIG. 12. The approach by the second embodiment enables evaluation of the cognitive function of a subject SJ based on respective features of a spatial positional relationship and temporal variations, of all joints of the subject SJ whose images have been captured, like the first embodiment. It is therefore possible to more accurately evaluate the cognitive function of the subject SJ, compared to a system that evaluates the cognitive function of a subject SJ based on limited features.

Note that although in the present embodiment, respective cognitive functions of subjects SJ are classified into the three classes, the cognitive function evaluation system 100 may classify respective cognitive functions of subjects SJ into four or more classes. For example, the cognitive function evaluation system 100 may classify subjects SJ into respective classes of types of dementia. The types of dementia include, for example, Alzheimer's type dementia, vascular dementia, Lewy body dementia, frontotemporal dementia, and normal pressure hydrocephalus. For example, the cognitive function evaluation system 100 may classify respective cognitive functions of subjects SJ into a class of Alzheimer's type dementia, a class of Lewy body dementia, a class of mild cognitive impairment, or a class of non-dementia. In this case, label data (teacher data) to be entered into a processor 73 (FIG. 7) during learning includes label data indicating Alzheimer's type dementia, label data indicating Lewy body dementia, label data indicating mild cognitive impairment, and label data indicating non-dementia.

Although in the present embodiment, the cognitive function of a subject SJ is classified into any of the three classes, the cognitive function evaluation system 100 may classify the cognitive function of a subject SJ into any of two classes. For example, the cognitive function evaluation system 100 may classify the cognitive function of a subject SJ into a class of dementia or a class of mild cognitive impairment and non-dementia, or into a class of dementia and mild cognitive impairment or a class of non-dementia, like the first embodiment.

The embodiments of the present invention is described above with reference to the accompanying drawings (FIGS. 1 to 12). The present invention may however be implemented in various manners within a scope not departing from the essence thereof and is not limited to the above embodiments. Furthermore, the constituent elements disclosed in the above-described embodiments may be altered as appropriate. For example, some constituent elements among all of the constituent elements illustrated in one embodiment may be added to the constituent elements of another embodiment, or some constituent elements among all of the constituent elements illustrated in one embodiment may be removed from the embodiment.

The drawings illustrate each constituent element mainly in a schematic manner to facilitate understanding of the invention. Aspects such as the thickness, length, number, and interval of each constituent element illustrated in the drawings may differ in practice for convenience of drawing preparation. It also need not be stated that the configuration of each constituent element illustrated in the above embodiments is an example and is not a particular limitation. In addition, various changes can be made without substantially deviating from the effects of the present invention.

For example, in the embodiments described with reference to FIGS. 1 to 12, a subject SJ is required to perform a cognitive task (single task), a physical task (single task), and a dual task in this order. The order in which the cognitive task (single task), the physical task (single task), and the dual task are performed is however interchangeable.

In the embodiments described with reference to FIGS. 1 to 12, a subject SJ is required to perform the cognitive task (single task), the physical task (single task), and the dual task in this order. A manner of requiring a subject SJ to perform tasks is however arbitrary. For example, a subject SJ may be required to perform a dual task, a cognitive task (single task), a physical task (single task), and a dual task in this order. Alternatively, a subject SJ may be required to perform a cognitive task (single task) and a dual task, or to perform a physical task (single task) and a dual task. Tasks to be required for a subject SJ may only be a dual task. In the case where a cognitive task (single task) and a dual task are performed by a subject SJ, the cognitive task (single task) and the dual task may be performed in any order. Similarly, in the case where a physical task (single task) and a dual task are performed by a subject SJ, the physical task (single task) and the dual task may be performed in any order.

In the embodiments described with reference to FIGS. 1 to 12, the task-presenting section 10 includes a display. The task-presenting section 10 may however include a sound output device.

In the embodiments described with reference to FIGS. 1 to 12, the answer detector 30 includes answer switches. The answer detector 30 is however not limited to the answer switches. Examples of the answer detector 30 may include a line-of-sight detector and a sound collector.

An approach using a line-of-sight detector makes it possible to get answers from a subject SJ based on the direction of his or her line-of-sight to an answer-candidate-presenting screen 12b as depicted in FIGS. 2B and 3. Known line-of-sight-detecting technologies can be employed for such a line-of-sight detector. Examples of the line-of-sight detector include a near-infrared LED and an image-capturing device. The near-infrared LED emits near-infrared radiation toward the eyes of the subject SJ. The image-capturing device captures images of the eyes of the subject SJ. Processors 42 and 73 analyze the images captured by the image-capturing device to detect the position of the pupils (direction of line-of-sight) of the subject SJ.

An approach using a sound collector makes it possible to get answers from a subject SJ based on his or her voice emitted toward an answer-candidate-presenting screen 12b as depicted in FIGS. 2B and 3, for example. Processors 42 and 73 can get answers from the subject SJ by, for example converting the speech from his or her voice into text data through a speech recognition process.

Note that in the case where the sound collector is employed, questions on a cognitive examination are not limited to questions that require a subject SJ to choose one of two possible answers. For example, the subject SJ may be required to answer calculation questions. Also, in the case of the sound collector, questions on a cognitive examination may be word-answer questions. Examples of the word-answer questions may include "Shiritori (Japanese word game) problems", "questions to list language (e.g., words) that begins with sound (letter) arbitrarily selected from the 41oujon (Japanese syllabary)", and "question to list language (e.g., words) that begin with any letter arbitrarily selected from alphabet".

In the embodiments described with reference to FIGS. 1 to 12, the data-collecting section 70 is used to generate

**EP 4 424 241 B1**

training data. The evaluator 40 may generate training data.

INDUSTRIAL APPLICABILITY

The present invention can be used for the diagnosis of dementia.

REFERENCE SIGNS LIST

| 2 | Motion-feature-extracting section |
|---|---|
| 2a | First motion-feature-extracting section |
| 2b | Second motion-feature-extracting section |
| 2c | Third motion-feature-extracting section |
| 20 | Motion detector |
| 30 | Answer detector |
| 40 | Evaluator |
| 100 | Cognitive function evaluation system |
| L | Loss function |
| NW | Neural network |
| P1 | Sensitivity |
| P2 | Specificity |
| SJ | Subject |

**Claims**

1. A cognitive function evaluation system (100), comprising:

a motion detector (20) that captures images of a subject (SJ) performing a predetermined task to generate frames representing a three-dimensional human skeleton model that moves according to motion of the subject (SJ) whose images have been captured, the frames being a series of frames generated in time order;
an answer detector (30) that detects answers to questions on a predetermined cognitive examination by the subject (SJ) performing the predetermined task; and
an evaluator (40) that outputs motion features based on the frames and evaluates, using a trained model (TM), a cognitive function of the subject (SJ) based on the motion features and the answers detected by the answer detector (30), the motion features representing a feature of a spatial positional relationship of joints included in the three-dimensional human skeleton model and features of respective temporal variations of each of the joints, wherein
the predetermined task includes

a physical task which is a single task and requires the subject (SJ) to perform a predetermined behavior, and
a cognitive task which is a single task and requires the subject (SJ) to answer the questions on the predetermined cognitive examination, and
a dual task which requires the subject (SJ) to perform the physical task and the cognitive task simultaneously,

the cognitive task, the physical task, and the dual task are performed continuously in a predetermined order,
the motion detector (20) captures the images of the subject (SJ) performing the physical task and the dual task to generate the frames, and
the answer detector (30) detects the answers by the subject (SJ) performing the cognitive task and the dual task.

2. The cognitive function evaluation system (100) according to claim 1, wherein the evaluator (40) classifies the cognitive function of the subject (SJ) into a class in which a cognitive function score indicating a cognitive ability of the subject (SJ) is less than or equal to a threshold or a class in which the cognitive function score is greater than the threshold.

3. The cognitive function evaluation system (100) according to claim 2, wherein according to the threshold that is set in advance, the evaluator (40) classifies the subject (SJ) into a class of dementia or a class of mild cognitive impairment and non-dementia, or into a class of dementia and mild cognitive impairment or a class of non-dementia.

17

4. A cognitive function evaluation system (100) according to any one of claims 1 to 3, wherein the evaluator (40) determines a cognitive function score indicating a cognitive ability of the subject (SJ).

5. The cognitive function evaluation system (100) according to claim 1, wherein the evaluator (40) classifies the subject (SJ) into a class of dementia, a class of mild cognitive impairment, or a class of non-dementia.

6. The cognitive function evaluation system (100) according to claim 5, wherein the evaluator (40) classifies the subject (SJ) into any one of at least two types of the dementia.

7. The cognitive function evaluation system (100) according to claim 1, wherein the evaluator (40) includes a motion feature extractor (2) that extracts the motion features by:

generating respective spatial graphs for the frames, each of the respective spatial graphs indicating respective spatial positional relationships of each of the joints included in the three-dimensional human skeleton model; convolving the respective spatial graphs; generating time graphs across the frames, each of the time graphs representing respective variations in an identical joint between each adjacent frames; and convolving the time graphs.

8. The cognitive function evaluation system (100) according to claim 7, wherein:

the evaluator (40) includes a plurality of motion feature extractors (2a, 2b, 2c) each of which corresponds to the motion feature extractor (2); each of the plurality of motion feature extractors (2a, 2b, 2c) is supplied with corresponding frames for each time the predetermined task is performed a plurality of times continuously; and the evaluator (40) evaluates the cognitive function of the subject (SJ) based on the motion features acquired from each of the plurality of motion feature extractors (2a, 2b, 2c) and the answers detected by the answer detector (30).

9. A computer-implemented method that determines parameter values for a neural network (NW) that classifies a subject (SJ) as positive or negative, the neural network (NW) being built by the trained model (TM) used in the cognitive function evaluation system according to any one of claims 1 to 8, wherein the computer-implemented method comprises determining the parameter values through a loss function that optimizes a sum of sensitivity and specificity, the sensitivity describing a rate of the subject (SJ) being identified as true positive, the specificity describing a rate of the subject (SJ) being identified as true negative.

10. A computer-implemented method that determines parameter values for a neural network (NW) for use in training the trained model (TM) used in the cognitive function evaluation system according to any one of claims 1 to 8, wherein

the neural network (NW) includes a first network (2a) and a second network (2b) that convolve spatial graphs and convolve time graphs, the spatial graphs representing respective spatial positional relationships of joints included in a three-dimensional human skeleton model that moves according to motion of a subject (SJ) whose images have been captured, the time graphs representing respective temporal variations of the joints included in the three-dimensional human skeleton model, and the computer-implemented method includes

(S1) determining parameter values of the first network (2a) by learning from data entered into the first network (2a), and (S2, S3) determining parameter values of the second network (2b) by learning from data entered into the second network (2b) after setting the determined parameter values of the first network (2a) as initial values of parameter values of the second network (2b).

**Patentansprüche**

1. Ein System zum Auswerten kognitiver Funktionen (100), das aufweist:

einen Bewegungsdetektor (20), der Aufnahmen eines Subjekts (SJ) erfasst, das eine vorbestimmte Aufgabe

EP 4 424 241 B1

ausführt, um Bilder zu erzeugen, die ein dreidimensionales menschliches Skelettmodell darstellen, das sich gemäß der Bewegung des Subjekts (SJ), dessen Aufnahmen erfasst wurden, bewegt, wobei die Bilder eine Reihe von Bildern sind, die in zeitlicher Reihenfolge erzeugt werden,

einen Antwortdetektor (30), der Antworten auf Fragen zu einer vorbestimmten kognitiven Untersuchung des Subjekts (SJ), das die vorbestimmte Aufgabe ausführt, detektiert, und

einen Auswerter (40), der Bewegungsmerkmale basierend auf den Bildern ausgibt und unter Verwendung eines trainierten Modells (TM) eine kognitive Funktion des Subjekts (SJ) basierend auf den Bewegungsmerkmalen und den mittels des Antwortdetektors (30) detektierten Antworten auswertet, wobei die Bewegungsmerkmale ein Merkmal einer räumlichen Positionsbeziehung von Gelenken, die in dem dreidimensionalen menschlichen Skelettmodell enthalten sind, und Merkmale jeweiliger zeitlicher Variationen jedes der Gelenke darstellen, wobei die vorbestimmte Aufgabe aufweist

eine physische Aufgabe, die eine einzelne Aufgabe ist und von dem Subjekt (SJ) verlangt, ein vorbestimmtes Verhalten auszuführen, und

eine kognitive Aufgabe, die eine einzelne Aufgabe ist und von dem Subjekt (SJ) verlangt, die Fragen der vorbestimmten kognitiven Untersuchung zu beantworten, und

eine Dualaufgabe, die von dem Subjekt (SJ) verlangt, die physische Aufgabe und die kognitive Aufgabe zugleich auszuführen,

die kognitive Aufgabe, die physische Aufgabe und die Dualaufgabe kontinuierlich in einer vorbestimmten Reihenfolge ausgeführt werden,

der Bewegungsdetektor (20) die Aufnahmen des Subjekts (SJ), das die physische Aufgabe und die Dualaufgabe ausführt, erfasst, um die Bilder zu erzeugen, und

der Antwortdetektor (30) die Antworten des Subjekts (SJ) erfasst, das die kognitive Aufgabe und die Dualaufgabe ausführt.

2. Das System zum Auswerten kognitiver Funktionen (100) gemäß Anspruch 1, wobei der Auswerter (40) die kognitiven Funktionen des Subjekts (SJ) in eine Klasse einteilt, in der ein kognitive-Funktion-Wert, der eine kognitive Fähigkeit des Subjekts (SJ) angibt, kleiner oder gleich einem Schwellenwert ist, oder in eine Klasse, in der der kognitive-Funktion-Wert größer als der Schwellenwert ist.

3. Das System zum Auswerten kognitiver Funktionen (100) gemäß Anspruch 2, wobei der Auswerter (40) das Subjekt (SJ) gemäß dem vorab festgelegten Schwellenwert in eine Klasse von Demenz oder eine Klasse von leichter kognitiver Beeinträchtigung und ohne Demenz, oder in eine Klasse von Demenz und leichter kognitiver Beeinträchtigung oder eine Klasse ohne Demenz einordnet.

4. Ein System zum Auswerten kognitiver Funktionen (100) gemäß irgendeinem der Ansprüche 1 bis 3, wobei der Auswerter (40) einen kognitive-Funktion-Wert ermittelt, der eine kognitive Fähigkeiten des Subjekts (SJ) angibt.

5. Das System zum Auswerten kognitiver Funktionen (100) gemäß Anspruch 1, wobei der Auswerter (40) das Subjekt (SJ) in eine Klasse von Demenz, eine Klasse von leichter kognitiver Beeinträchtigung oder eine Klasse ohne Demenz einordnet.

6. Das System zum Auswerten kognitiver Funktionen (100) gemäß Anspruch 5, wobei der Auswerter (40) das Subjekt (SJ) in irgendeine von mindestens zwei Arten von Demenz einordnet.

7. Das System zum Auswerten kognitiver Funktionen (100) gemäß Anspruch 1, wobei der Auswerter (40) einen Bewegungsmerkmalextraktor (2) aufweist, der die Bewegungsmerkmale extrahiert, mittels

Erzeugens jeweiliger räumlicher Graphen für die Bilder, wobei jeder der jeweiligen räumlichen Graphen jeweilige räumliche Positionsbeziehungen jedes der Gelenke angibt, die in dem dreidimensionalen menschlichen Skelettmodell enthalten sind,
Faltens der jeweiligen räumlichen Graphen,
Erzeugens von Zeitgraphen über die Bilder hinweg, wobei jeder der Zeitgraphen jeweilige Variationen in einem identischen Gelenk zwischen jedem der benachbarten Bilder darstellt, und
Faltens der Zeitgraphen.

8. Das System zum Auswerten kognitiver Funktionen (100) gemäß Anspruch 7, wobei:

der Auswerter (40) eine Mehrzahl von Bewegungsmerkmalextraktoren (2a, 2b, 2c) aufweist, von denen jeder dem Bewegungsmerkmalextraktor (2) entspricht,

jeder der Mehrzahl von Bewegungsmerkmalextraktoren (2a, 2b, 2c) mit entsprechenden Bildern versorgt wird für jedes Mal, wenn die vorbestimmte Aufgabe eine Mehrzahl von Malen kontinuierlich ausgeführt wird, und der Auswerter (40) die kognitive Funktion des Subjekts (SJ) basierend auf den von jedem der Mehrzahl von Bewegungsmerkmalextraktoren (2a, 2b, 2c) erfassten Bewegungsmerkmale und den mittels des Antwortdetektors (30) erfassten Antworten auswertet.

9.  Ein computerimplementiertes Verfahren, das Parameterwerte für ein neuronales Netzwerk (NW) ermittelt, das ein Subjekt (SJ) als positiv oder negativ klassifiziert, wobei das neuronale Netzwerk (NW) mittels des trainierten Modell (TM) aufgebaut wird, das in dem System zum Auswerten kognitiver Funktionen gemäß irgendeinem der Ansprüche 1 bis 8 verwendet wird, wobei das computerimplementierte Verfahren Ermitteln der Parameterwerte durch eine Verlustfunktion, die eine Summe aus Sensitivität und Spezifität optimiert, aufweist, wobei die Sensitivität eine Rate beschreibt, dass das Subjekt (SJ) als echt positiv identifiziert wird, wobei die Spezifität eine Rate beschreibt, dass das Subjekt (SJ) als echt negativ identifiziert wird.

10. Ein computerimplementiertes Verfahren, das Parameterwerte für ein neuronales Netzwerk (NW) zur Verwendung beim Training des trainierten Modells (TM) ermittelt, das in dem System zum Auswerten kognitiver Funktionen gemäß irgendeinem der Ansprüche 1 bis 8 verwendet wird, wobei

das neuronale Netzwerk (NW) ein erstes Netzwerk (2a) und ein zweites Netzwerk (2b) aufweist, die räumliche Graphen falten und zeitliche Graphen falten, wobei die räumlichen Graphen jeweilige räumliche Positionsbeziehungen von Gelenken darstellen, die in einem dreidimensionalen menschlichen Skelettmodell enthalten sind, das sich gemäß Bewegung eines Subjekts (SJ), dessen Aufnahmen erfasst wurden, bewegt, wobei die zeitlichen Graphen jeweilige zeitliche Variationen der Gelenke darstellen, die in dem dreidimensionalen menschlichen Skelettmodell enthalten sind, und das computerimplementierte Verfahren aufweist

(S1) Ermitteln von Parameterwerten des ersten Netzwerks (2a) mittels Lernens aus Daten, die in das erste Netzwerk (2a) eingegeben wurden, und
(S2, S3) Ermitteln von Parameterwerten des zweiten Netzwerks (2b) mittels Lernens aus Daten, die in das zweite Netzwerk (2b) eingegeben wurden, nachdem die ermittelten Parameterwerte des ersten Netzwerks (2a) als Anfangswerte von Parameterwerten des zweiten Netzwerks (2b) festgelegt wurden.

**Revendications**

1.  Système d'évaluation de fonctions cognitives (100), comprenant :

un détecteur de mouvement (20) qui capture des images d'un sujet (SJ) effectuant une tâche prédéterminée afin de générer des images représentant un modèle de squelette humain tridimensionnel qui se déplace en fonction des mouvements du sujet (SJ) dont les images ont été capturées, les images étant une série d'images générées dans l'ordre chronologique ;
un détecteur de réponses (30) qui détecte les réponses aux questions d'un examen cognitif prédéterminé par le sujet (SJ) effectuant la tâche prédéterminée ; et
un évaluateur (40) qui émet des caractéristiques de mouvement sur la base des images et évalue, à l'aide d'un modèle entraîné (TM), une fonction cognitive du sujet (SJ) sur la base des caractéristiques de mouvement et des réponses détectées par le détecteur de réponses (30), les caractéristiques de mouvement représentant une caractéristique d'une relation spatiale de position des articulations incluses dans le modèle de squelette humain tridimensionnel et des caractéristiques de variations temporelles respectives de chacune des articulations, dans lequel
la tâche prédéterminée comprend

une tâche physique qui est une tâche unique et qui exige que le sujet (SJ) effectue un comportement prédéterminé, et
une tâche cognitive qui est une tâche unique et qui exige que le sujet (SJ) réponde aux questions de l'examen cognitif prédéterminé, et
une double tâche qui exige que le sujet (SJ) effectue simultanément la tâche physique et la tâche cognitive,

la tâche cognitive, la tâche physique et la double tâche sont exécutées de manière continue dans un ordre prédéterminé,

le détecteur de mouvement (20) capture les images du sujet (SJ) exécutant la tâche physique et la double tâche afin de générer les images, et

le détecteur de réponses (30) détecte les réponses du sujet (SJ) exécutant la tâche cognitive et la double tâche.

2. Système d'évaluation des fonctions cognitives (100) selon la revendication 1, dans lequel l'évaluateur (40) classe la fonction cognitive du sujet (SJ) dans une classe dans laquelle un score de fonction cognitive indiquant une capacité cognitive du sujet (SJ) est inférieur ou égal à un seuil ou dans une classe dans laquelle le score de fonction cognitive est supérieur au seuil.

3. Système d'évaluation des fonctions cognitives (100) selon la revendication 2, dans lequel, en fonction du seuil défini à l'avance, l'évaluateur (40) classe le sujet (SJ) dans une classe de démence ou une classe de troubles cognitifs légers et de non-démence, ou dans une classe de démence et de troubles cognitifs légers ou une classe de non-démence.

4. Système d'évaluation des fonctions cognitives (100) selon l'une quelconque des revendications 1 à 3, dans lequel l'évaluateur (40) détermine un score de fonction cognitive indiquant une capacité cognitive du sujet (SJ).

5. Système d'évaluation des fonctions cognitives (100) selon la revendication 1, dans lequel l'évaluateur (40) classe le sujet (SJ) dans une classe de démence, une classe de troubles cognitifs légers ou une classe de non-démence.

6. Système d'évaluation des fonctions cognitives (100) selon la revendication 5, dans lequel l'évaluateur (40) classe le sujet (SJ) dans l'un quelconque d'au moins deux types de démence.

7. Système d'évaluation des fonctions cognitives (100) selon la revendication 1, dans lequel l'évaluateur (40) comprend un extracteur de caractéristiques de mouvement (2) qui extrait les caractéristiques de mouvement en :

générant des graphes spatiaux respectifs pour les images, chacun des graphes spatiaux respectifs indiquant des relations spatiales respectives de chacune des articulations incluses dans le modèle de squelette humain tridimensionnel ;
convoluant les graphes spatiaux respectifs ;
générant des graphes temporels à travers les images, chacun des graphes temporels représentant des variations respectives dans une articulation identique entre chaque image adjacente ; et
en convoluant les graphes temporels.

8. Système d'évaluation des fonctions cognitives (100) selon la revendication 7, dans lequel :

l'évaluateur (40) comprend une pluralité d'extracteurs de caractéristiques de mouvement (2a, 2b, 2c) dont chacun correspond à l'extracteur de caractéristiques de mouvement (2) ;
chacun de la pluralité d'extracteurs de caractéristiques de mouvement (2a, 2b, 2c) est alimenté en des images correspondantes pour chaque fois que la tâche prédéterminée est effectuée plusieurs fois de manière continue ; et
l'évaluateur (40) évalue la fonction cognitive du sujet (SJ) sur la base des caractéristiques de mouvement acquises à partir de chacun de la pluralité d'extracteurs de caractéristiques de mouvement (2a, 2b, 2c) et des réponses détectées par le détecteur de réponses (30).

9. Procédé mis en œuvre par ordinateur qui détermine des valeurs de paramètres d'un réseau neuronal (NW) qui classe un sujet (SJ) comme positif ou négatif, le réseau neuronal (NW) étant construit par le modèle entraîné (TM) utilisé dans le système d'évaluation des fonctions cognitives selon l'une quelconque des revendications 1 à 8, dans lequel le procédé mis en œuvre par ordinateur comprend la détermination des valeurs de paramètres par le biais d'une fonction de perte qui optimise une somme de sensibilité et de spécificité, la sensibilité décrivant un taux du sujet (SJ) identifié comme vrai positif, la spécificité décrivant un taux du sujet (SJ) identifié comme vrai négatif.

10. Procédé mis en œuvre par ordinateur qui détermine des valeurs de paramètres pour un réseau neuronal (NW) destiné à être utilisé dans l'entraînement du modèle entraîné (TM) utilisé dans le système d'évaluation des fonctions cognitives selon l'une quelconque des revendications 1 à 8, dans lequel

le réseau neuronal (NW) comprend un premier réseau (2a) et un deuxième réseau (2b) qui convoluent des graphes spatiaux et convoluent des graphes temporels, les graphes spatiaux représentant les relations spatiales respectives de positions d'articulations incluses dans un modèle de squelette humain tridimensionnel qui se déplace en fonction du mouvement d'un sujet (SJ) dont les images ont été capturées, les graphes temporels représentant des variations temporelles respectives des articulations incluses dans le modèle de squelette humain tridimensionnel, et

le procédé mis en œuvre par ordinateur comprend :

(S1) la détermination de valeurs de paramètres du premier réseau (2a) par apprentissage à partir de données entrées dans le premier réseau (2a), et

(S2, S3) la détermination de valeurs de paramètres du deuxième réseau (2b) par apprentissage à partir de données entrées dans le deuxième réseau (2b) après avoir défini les valeurs de paramètres déterminées du premier réseau (2a) comme valeurs initiales de valeurs de paramètres du deuxième réseau (2b).

100

30

Answer detector

SJ

21

20

22

Motion-capturing section

Evaluator

40

Task-presenting section

10

# FIG. 1

FIG. 2A

FIG. 2B

FIG. 2C

FIG. 2D

12a · 10 · 10 · 12b · 10

| Memorize position<br><br>● |  | Was position here?<br><br>Yes ● No |
| --- | --- | --- |
| Present question | Remove question | Present answer candidates |

## FIG. 3

100

40

30 — Answer detector

20 — Motion detector

10 — Task-presenting section

50 — Identification-information-acquiring section

Processor

Storage

41 — Trained model — TM

42

Evaluator

60 — Output section

## FIG. 4

FIG. 5

30
Answer detector

20
Motion detector

10
Task-presenting section

50
Identification-information -acquiring section

70
Data-collecting section

Training data

200

40
Evaluator

FIG. 6

200

30
Answer detector

20
Motion detector

10
Task-presenting section

50
Identification-information -acquiring section

70

Processor

Input section — 71

Storage — 72

73

Data-collecting section

FIG. 7

200

40

·Loss function
·Learning rate
·Dropout value

42

Training data ——→ Processor

(Mini-batch)

Input section

43

41

TP

Training
program

Storage

Evaluator

FIG. 8

FIG. 9

NW, TM

First frame group → First motion-feature-extracting section (ST-GCN) [2, 2a] → First transforming section (FC) [3, 3a]

Parameter values → Second motion-feature-extracting section (ST-GCN) [2, 2b]

Second frame group → Second motion-feature-extracting section (ST-GCN) → Second transforming section (FC) [3, 3b]

Parameter values → Third motion-feature-extracting section (ST-GCN) [2, 2c]

Third frame group → Third motion-feature-extracting section (ST-GCN) → Third transforming section (FC) [3, 3c]

Answer feature data → Convolving section (CNN) [5]

Combining section (FC) [4]

Combining section (FC) [6] → Scalar value

Scalar value → Score-determining section (FC) [7] → Cognitive function score

```
┌─────────────────────────────────┐
│  Determine parameter values of  │ ⌐S1
│  first motion-feature-extracting │
│            section              │
└─────────────────────────────────┘
              │
              ▼
┌─────────────────────────────────┐
│ Set initial values of parameter values │ ⌐S2
│ of second motion-feature-extracting │
│            section              │
└─────────────────────────────────┘
              │
              ▼
┌─────────────────────────────────┐
│  Determine parameter values of  │ ⌐S3
│ second motion-feature-extracting │
│            section              │
└─────────────────────────────────┘
              │
              ▼
┌─────────────────────────────────┐
│   Set initial values of parameter   │ ⌐S4
│         values of third         │
│  motion-feature-extracting section  │
└─────────────────────────────────┘
              │
              ▼
┌─────────────────────────────────┐
│  Determine parameter values of  │ ⌐S5
│  third motion-feature-extracting │
│            section              │
└─────────────────────────────────┘
```

# FIG. 10

NW, TM

Frame group → Motion-feature
-extracting section
(ST-GCN) [2] → Transforming section
(FC) [3]

Scalar value

Combining section
(FC) — 6

Answer feature data

Scalar value

Score-determining
section
(FC)

7

Cognitive function
score

**FIG. 11**

NW, TM

First frame group → First motion-feature -extracting section (ST-GCN) 2, 2a

Second frame group → Second motion-feature -extracting section (ST-GCN) 2, 2b

Third frame group → Third motion-feature -extracting section (ST-GCN) 2, 2c

Answer feature data → Convolving section (CNN) 5

Combining section (FC) 4

Combining section (FC) 6a → Output

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018078184 A1
- US 2015208975 A1
- US 2020405216 A1
- CN 112070027 A

**Non-patent literature cited in the description**

- **TAKU MATSUURA**. Cognitive function score estimation for elderly people based on dual-task gait analysis. *IECE, Technical Research Report, Kawasaki*, March 2020, vol. 119 (HCS2019-99), 83-88